# EUROPEAN PATENT APPLICATION

(11) **EP 4 781 908 A2**
(43) Date of publication of application: **29.07.2026**
(21) Application number: 26176669.5
(22) Date of filing: 27.07.2023
(51) Int. Cl.: A61B 5/11

(54) **MONITORING SLEEPING DISORDERS AND SYSTEM THEREOF**

(30) Priority: 27.07.2022 EP 22187174
(62) Divisional of application: 23749007.3
(71) Applicant: Fundació Institut de Bioenginyeria de Catalunya (IBEC), 08028 Barcelona (ES); Universitat politècnica de Catalunya (UPC), 08034 Barcelona (ES)
(72) Inventor: JANÉ CAMPOS, Raimon, 08027 Barcelona (ES); FERRER LLUÍS, Ignasi, 17003 Girona (ES); CASTILLO ESCARIO, Yolanda, 08032 Barcelona (ES)
(74) Representative: Hoffmann Eitle

(57) **Abstract**

A system for monitoring sleeping quality in a subject person, the system comprising: a mobile device comprising a microphone configured to acquire audio signals indicative of breathing of the subject person and an accelerometer configured to acquire accelerometer signals including position, angular and movement information of the subject person; a fixation system for collocating the mobile device with the subject person; and a pulse oximeter configured to measure oxygen saturation levels of the subject person, wherein the fixation system is composed of an elastic band and a bag to place the mobile device; and wherein the system is configured for monitoring sleeping quality in a subject person.

## Description

### Technical Field

The present invention relates to a method for monitoring a sleeping disorder and a sleeping quality in a subject person. More particular, the present invention relates to corresponding method and systems that employ mobile devices, such as smartphones or other wearable devices. Some aspects may also relate to corresponding positional treatment.

### Background Art

Obstructive sleep apnea (OSA) is a prevalent sleep disorder that is related to sleepiness and accidents and is also an independent risk factor of cardiac, brain and cancer diseases. However, more than 80% of patients are late diagnosed, as conventional techniques to diagnose OSA do not provide a method for monitoring sleeping of a subject person in a reliable and also in a cost-effective way. Known methods are based on questionnaires, such as the STOP-Band and Berlin questionnaires, which are supposed to quantify the sleep quality of the patient. However, they oftentimes fail to properly determine different sleeping positions and find their relations to the sleep apnea.

Other techniques, such as polysomnography (PSG), which some regard as the golden-standard test for diagnosing sleep-related diseases, use multiple sensors, such as a nasal pressure transducer, thermistor, and thoracic and abdominal bands, for recording breathing activity, and accelerometry-based sensors, as well as cameras, for monitoring sleep position, which leads to a more objective and reliable measure. However, the PSG is cumbersome and costly; it requires the subject to sleep with multiple wires, and promotes the subject to sleep in a certain supine position, which may even worsen any disorder already present.

In addition to the above, it is well known to employ the today ubiquitous presence of mobile electronic devices, especially smartphones. The capabilities of modern smartphones have long been appreciated in many technology fields. The fact that such mobile devices do not only provide substantial processing power and high-quality user interfaces, but also sensors and detectors for sound, light, position, accelerations and forces and the like, has already led to a broad variety of applications in technical fields that do not directly relate to telephony and communication.

However, the mere existence of sensors and data acquisition and processing capabilities does not automatically yield practicable solutions. Especially in the context of health-related issues, in the field of telemedicine, telehealth, telemonitoring and mHealth, there is the need for a factual provision of well-defined, predictable and - above all - reliable solutions and related advantages.

There is therefore a need for improved methods and systems that provide reliable functionality in the context of sleeping disorders.

### Summary

The above problems are solved by the subject-matter of the independent claims. Further preferred embodiments are given by the subject-matter of the dependent claims. Moreover, further examples are provided for facilitating the understanding of the invention.

According to an embodiment of the present invention, there is provided a method for monitoring a sleeping disorder in a subject person, the using a mobile device and comprising: collocating the mobile device with the subject person; acquiring audio signals indicative of breathing of the subject person by means of a microphone of the mobile device; acquiring accelerometer signals, by means of an accelerometer of the mobile device, to derive position, angular and movement information of the subject person from the accelerometer signals or the signals from the accelerometer; measuring thoracic movement related to respiration and sleep-disordered breathing by means of the accelerometer; identifying silence events based on the audio signals; classifying the silence events into apneas or hypopneas; identifying an apnea type of the classified apnea based on the thoracic movement; and associating to a classified silence event the respective angular information by combining the audio signals and the accelerometer signals.

According to another embodiment of the present invention, there is provided a method for monitoring a sleeping quality in a subject person, the method using a mobile device and comprising: collocating the mobile device with the subject person; acquiring audio signals indicative of breathing of the subject person by means of a microphone of the mobile device; acquiring accelerometer signals, by means of an accelerometer of the mobile device, to derive position, angular and movement information of the subject person from the accelerometer signals; measuring thoracic movement related to respiration and sleep-disordered breathing by means of the accelerometer; identifying a sleep event based on the audio signals; and identifying body position at which the sleep event is produced based on the angular information.

According to another embodiment of the present invention, there is provided a system for monitoring sleeping disorder and quality in a subject person, the system comprising: a mobile device comprising; a microphone configured to acquire audio signals indicative of breathing of the subject person; and an accelerometer configured to acquire accelerometer signals including position, angular and movement information of the subject person, a fixation system for collocation the mobile device with the subject person; and a pulse oximeter configured to measure oxygen saturation levels of the subject person, wherein the fixation system is composed of an elastic band and a bag to place the mobile device.

### Brief Description of the Drawings

Embodiments of the present invention, which are presented for better understanding the inventive concepts, but which are not to be seen as limiting the invention, will now be described with reference to the figures in which:
- Figure 1: shows a schematic view of a configuration for applying and embodiment of the present invention;
- Figure 2: shows schematically the concepts of the position, angular and movement information related to a subject person in embodiments of the present invention;
- Figure 3: shows a schematic view of a configuration of the fixation system which is for collocating the mobile device with the subject person;
- Figure 4: shows a schematic view of a configuration of the system for monitoring sleeping disorder and quality in a subject person;

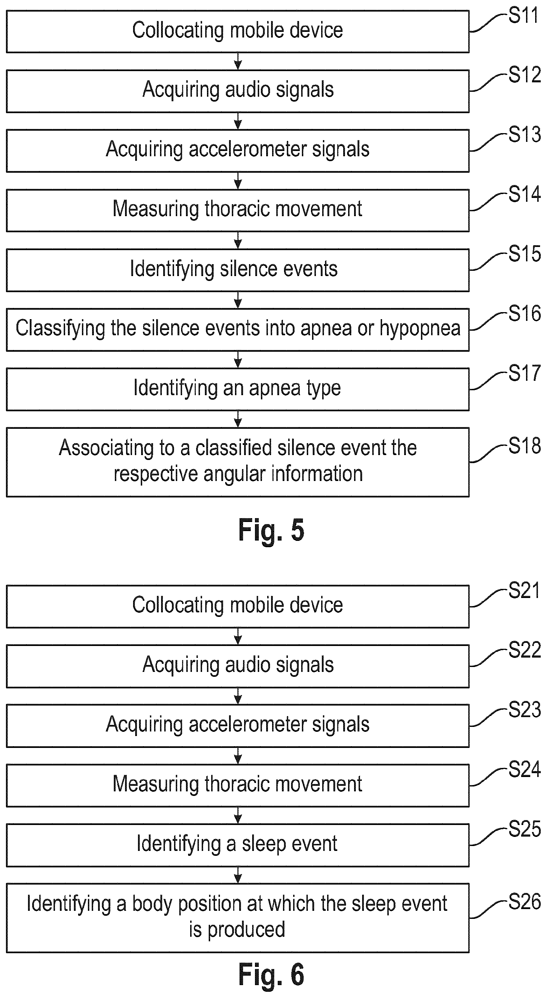

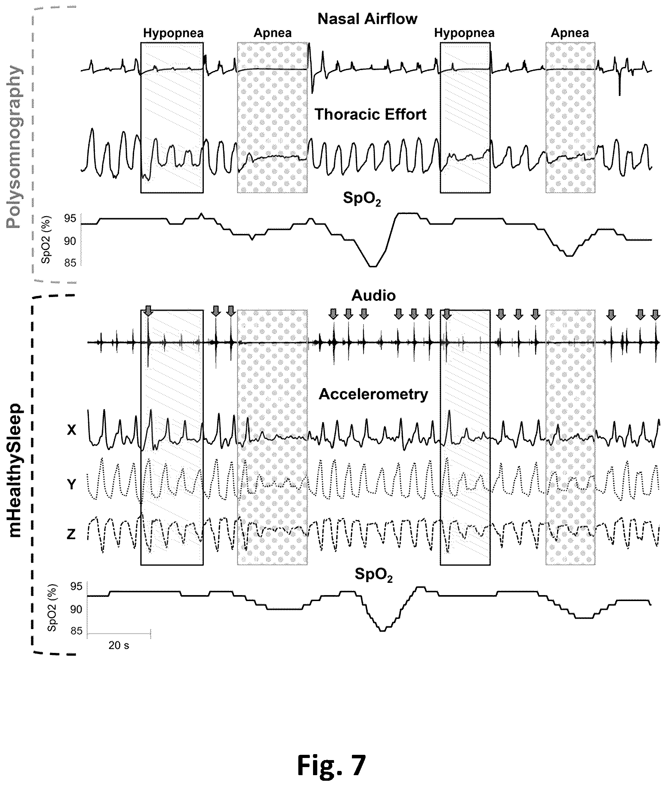

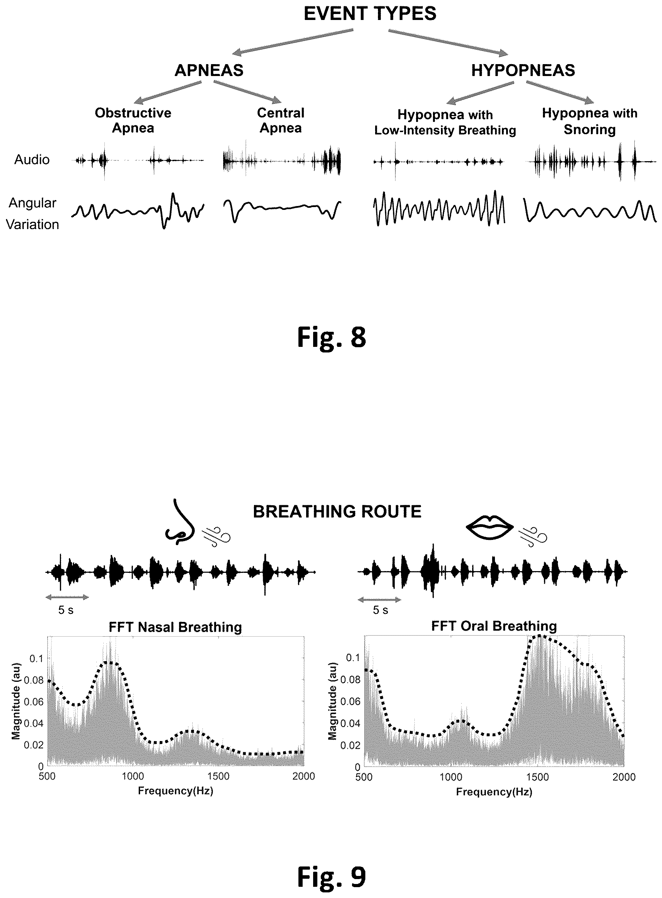

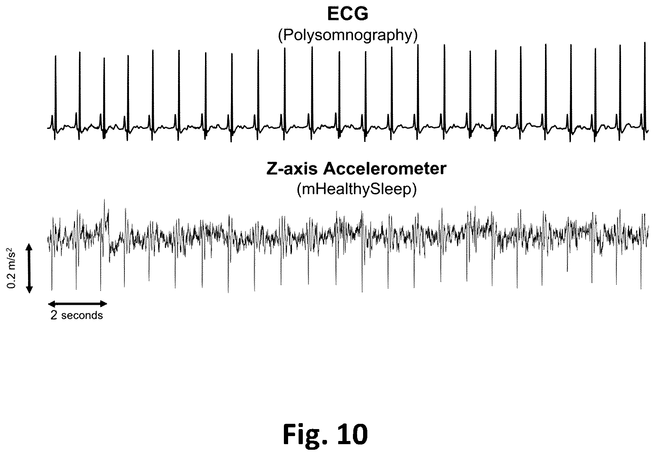

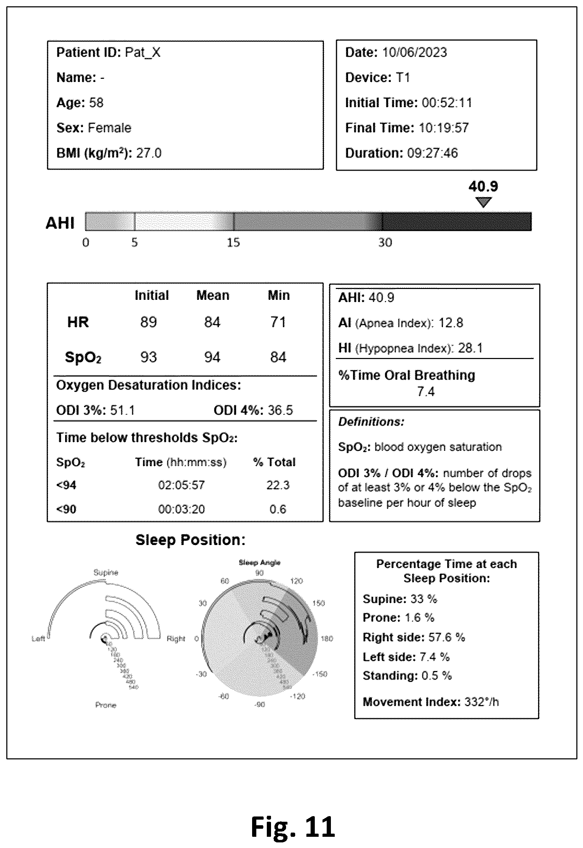

- Figure 5: shows a schematic flow chart of a general method embodiment of the present invention; and
- Figure 6: shows a schematic flow chart of a method for monitoring sleeping quality of the present invention.

### Detailed Description

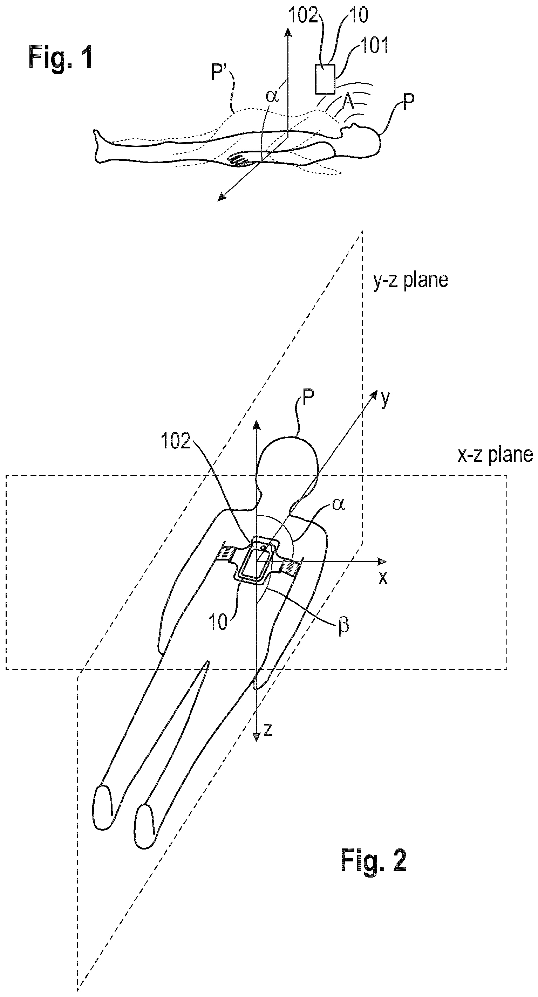

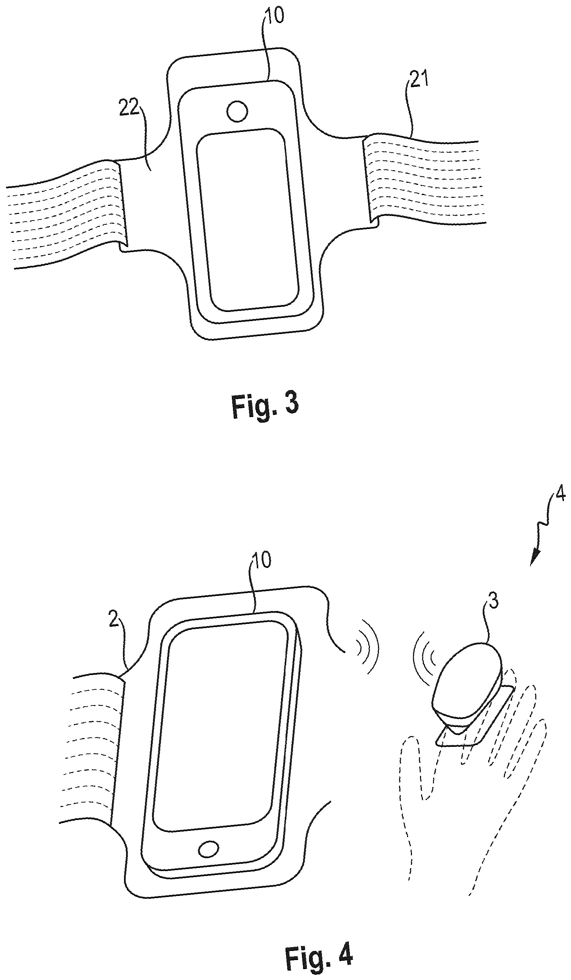

Figure 1 shows a schematic view of a configuration for applying and embodiment of the present invention. Specifically, this embodiment provides monitoring a sleeping disorder in a subject person P and the configuration considers a mobile device 10 and collocating the mobile device 10 with the subject person P. Said collocating may comprise ensuring that the mobile device 10 follows the movements by the subject person P. For example, the collocating can be obtained by securing the mobile device 10 in a pocket of apparel worn by the subject person P. Further, the collocating may be obtained by means of a fixation system 2 affixed by a strip or belt to the subject person P. The fixation system 2 is further detailed in Figure 3. Generally, said mobile devices can be implemented by any one of a mobile computing and/or communication device, a mobile phone, a smartphone, a wearable device, an electronic wristwatch, a smartwatch, and the like.

The embodiment then comprises acquiring audio signals. The audio signals indicative of breathing of the subject person P are acquired by means of a microphone 101 of the mobile device 10. For example, the audio signals A may be obtained by a built-in microphone 101 of the mobile device 10 during the overnight, with a sampling rate of 48 kHz.

The embodiment further comprises acquiring accelerometer signals, by means of an accelerometer 102 of the mobile device 10, to derive position, angular (e.g. as depicted with α in Fig. 1) and movement information of the subject person P from the accelerometer signals. As the mobile device 10 is collocated with the subject person P, the mobile device's accelerometer 102 can advantageously detect the subject person's movement while monitoring an acute or potential sleeping disorder. Specifically, the accelerometer signals can be employed for detecting or distinguishing a supine position (as shown in Figure 1 for the subject person P or a lateral position indicated by P' in Figure 1. Although not shown in Figure 1, the accelerometer signals can also be employed for detecting or distinguishing other positions such as a prone or an upright position of the subject person P. Furthermore, a sleeping position of the subject person P is not limited to one of the pure supine, lateral, prone or upright position where the angular information assumes roughly multiples of 90°, and the sleeping position of the subject person P is not limited to the supine position. The angular information may be an angle, a plurality of angles, or triaxial angles. The position, angular and movement information related to a subject person are further detailed in conjunction with Figure 2.

The present embodiment further comprises measuring thoracic movement related to respiration and sleep-disordered breathing by means of the accelerometer 102. This means, for example, the accelerometer 102 of the mobile device 10 collocated above the sternum of the subject person P may be used to measure repetitive vertical movements of the chest caused by breathing of the subject person P. When breathing is present in a subject person, it is possible recognize the existence of the breathing by movements of the chest as the anterior-posterior, vertical and transverse dimensions of the thorax are increased and deceased in inhalation and exhalation each. The collocated mobile device 10 can be used in measuring this thoracic movement related to respiration.

Further, when the respiration is paused or weakened due to the sleeping disorder, the sleep-disordered breathing can be also detected as the movement of the chest may be temporarily stopped or weakened.

The present embodiment further comprises identifying silence events based on the audio signals. The silence events can correspond to events which may represent sleeping disorder. For example, when the subject person P has OSA, there may be repetitive episodes of total or partial airflow reduction during sleep (i.e., apneas and hypopneas), produced by upper airway obstruction. These breathing disturbances may be recognized as silence events in the audio signals A. For the recognition of the silence events, sample entropy may be used in the calculation, as snoring and breathing present more complex patterns than silence regions, hence producing higher sample entropy values. However, the calculation used in the recognition of the silence events is not limited to a certain function.

Once the silence events are identified, the silence events are classified in apnea or hypopnea. In analysis of the audio signals A, if there is residual breathing activity present in the silence event, then the silence event would be classified as an event representing hypopnea, while if there was no breathing in the silence event at all, the silence event would be labelled as apnea. The residual breathing activity may be recognized by existence of low-intensity respiratory sounds recorded through the microphone 101.

The present embodiment further comprises identifying an apnea type of the classified apnea based on the thoracic movement. The accelerometer 102 may be used to identify the classified apnea into three types, which are, obstructive, central and mixed. The obstructive apnea is caused due to the upper way obstruction while the central apnea is produced when the brain does not send proper signals to the responsible muscles to control breathing. The mixed apnea is an apnea which begins as a central apnea and ends as an obstructive apnea or vice versa.

Further, the embodiment comprises associating to a classified silence event the respective angular information by combining the audio signals and the accelerometer signals. At each classified silence event, the respective angular information may be associated and be analysed to determine the positional occurrence of the sleeping disorder. Identification of the position represented by the angular information at the silent event which may represent apnea or hypopnea may be greatly helpful in some cases. For example, supine sleeping position is known to promote the occurrence of apnea compared to lateral and prone sleeping positions. This phenomenon is known as positional obstructive sleep apnea (pOSA) and is one of the most common diseases affecting sleeping quality. Therefore, revealed positional patterns of the sleeping disorder may be effectively used in monitoring a subject person with pOSA.

Figure 2 shows schematically the concepts of the position, angular information and movement information related to a subject person in embodiments of the present invention. Specifically, Figure 2 depicts a sleeping angle α and stand angle β according to x, y and z axes. As shown in Figure 2, the accelerometer 102 provides positive values when the acceleration goes from right to left (x-axis), from toe to head (y-axis) and from front to back (z-axis), thus defines the X-Z plane, where the sleep angle is calculated, and the Y-Z plane, where the stand angle is calculated. Sleeping angle α = 0° represents a pure left sleeping position, α = 90° represents a pure supine sleeping position, α = ± 180° represents pure right sleeping position and α = -90° represent pure prone sleeping position. Further, when the stand angle β = 0°, it means the position of the subject person P is headstand, a pure laying position when β = ± 90° and a standing position when β = ± 180°. Moreover, the thoracic movement caused by inhalation and exhalation of the subject person P may be explained in the Figure 2 as well. By measuring vertical movement, which may be represented by repetitive ups and downs in z-direction of the accelerometer 102 of the mobile device 10, or in other directions when the subject person P is sleeping in other positions, the thoracic movement of the subject person P may be recorded and further used in the classification of apnea types or in other applications.

Figure 3 shows a schematic view of a configuration of the fixation system 2 which is for collocating the mobile device 10 with the subject person P. Specifically, the mobile device 10 may be located in a bag 22 and attached to chest of the subject person P, or specifically over the sternum of the subject person P, with an elastic band 21.

In a further embodiment, the method comprises quantifying sleep movements and sleep position variability based on the accelerometer signals. For example, angular information derived from the accelerometer signals may be quantified as angular resolution related to the sleeping position. Further, position and/or movement information included in the accelerometer signals may be quantified as sleep position variability.

Moreover, the method further comprises detecting breathing route during sleep from the audio signals. The breathing route during sleep may be classified into oral breathing or nasal breathing. Oral breathing indicates that the subject person is breathing through the mouth whereas nasal breathing indicates that the subject person is breathing through the nose. Considering that oral breathing may be more prevalent with subjects with OSA, who have nasal obstruction that lead to increased breathing through the mouth, detecting breathing route by means of the audio signals A acquired by the mobile device 10 may be advantageous in monitoring sleeping disorder.

When the audio signal is acquired, the audio signal may be downsampled to 5 kHz, band-pass filtered between 70 and 2000 Hz and the power-line noise may be removed. If the signal is too noisy, an optimal filter or spectral subtraction may be applied to reduce interferences and improve signal quality. After that, using temporal, spectral, and time-frequency representations, apnea and hypopnea events may be identified. The spectrogram may be used as time-frequency representation with a window of 0.1s, 90% overlap and NFFT = 1024, to distinguish if there is any breathing present during the events and thus classify them into apnea or hypopnea. The Fast Fourier Transform may be calculated, and a linear envelope may be extracted, using windows of 15 Hz, to check if there is a prominent peak between 950 Hz and 2 kHz, indicative of oral breathing. The information extracted in this calculation may be used in further classification of hypopnea into nasal or oral breathing, and even in detecting the breathing route at any time point during the night. The percentage of time that the subject is breathing through the mouth can be calculated.

In yet another embodiment, the method further comprises acquiring oxygen saturation levels of the subject person P by means of a pulse oximeter 3 via a wireless link to the mobile device 10. The pulse oximeter is a non-invasive device for measuring blood oxygen saturation (SpO2). Apnea and hypopnea may be associated with local reductions in SpO2, which are desaturations followed by reoxygenations. For this reason, the oxygen saturation levels may be used as a measure for identifying OSA.

Moreover, the embodiment further comprises selecting regions of interest, wherein the regions of interest are regions in the audio signals A that are followed by oxygen desaturations measured by the wireless pulse oximeter 3. When the oxygen saturation level drops more than 3%, it may be recognized as a desaturation event and used in analysis of sleeping disorder such as apnea, hypopnea and/or snoring. For a higher accuracy, the silence events may be identified only if they are followed by desaturation events measured by the wireless pulse oximeter 3 that is coupled to the mobile device 10.

In one embodiment, the method further comprises removing artefacts by considering the accelerometer signals while identifying silence events. When the subject person P moves during sleep, the noise generated during the movements may be recorded with the breathing sound in the audio signals A. Therefore, the accelerometer 102 may be used to identify movements of the subject person P and remove artefacts in identifying silence events from the audio signals A.

In one embodiment, the method further comprises classifying the silence events into apneas or hypopneas depending on existence of low-intensity respiratory sounds in the audio signals. If the low-intensity respiratory sounds exists, it can be assumed that residual breathing activity is present and the respective silence event should be classified as a hypopnea. On the other hand, if there is no breathing at all, the silence event should be labelled as an apnea.

In another embodiment, the method further comprises providing the classified silence events with associated the respective angular information on the mobile device 10. The classified silence event that are associated which the respective angular information may be provided on the mobile device 10. Specifically, the classified silence events that are recorded during night may be shown in a smartphone application for an easier sleeping monitoring of the subject person P.

In another embodiment, the method further comprises outputting vibration and/or sound from the mobile device 10 based on the angular information and the oxygen saturation level and/or the audio signals. For example, when a subject person P using the mobile device 10 with the method provided in the embodiment, the method may be able to provide an alert to the subject person P in a specific position, such as supine position, which may promote occurrence of apnea compared to lateral and prone sleeping positions. For this positional treatment, the mobile device 10 is configured to output vibration and/or sound to alert the subject person P. By promoting the subject person P to change the sleeping position, sleeping in the supine position may be avoid by the positional treatment.

In one embodiment, the method further comprises detecting cardiac activity from the accelerometer 102. For example, the cardiac activity detected from the accelerometer 102 may include detection of changes of heart rate of the subject person. Then, the method further comprises associating a relationship of the changes of heart rate of the subject person and the silence events.

The invention may be used to monitor sleeping quality of the subject person P as well. The monitoring of the sleeping quality may be identified similarly to the monitoring of the sleeping disorders. In this embodiment, however, sleep events may be identified instead of the silence events.

For example, the method according to the embodiment comprises the same steps as in the method for monitoring a sleeping disorder until the identification of the sleep event. Then, the method according to the embodiment identifies a sleep event, including snoring episodes, based on audio signals A acquired by means of the microphone 101 of the mobile device 10.

Once the sleep event is identified, a body position at which the sleep event is produced is identified based on the angular information of the subject person P, which is derived from the accelerometer signals.

The embodiment further comprises automatic detecting of multimodal features and labelling of the sleep event by multimodal features. The multimodal features are derived from the audio signals A, the accelerometer signals and the oxygen saturation levels.

The embodiment further comprises detecting cardiac activity from the accelerometer 102 of the mobile device 10. The accelerometer signals automatically detected as a part of the multimodal features may contain higher frequency components compared to the movements from the accelerometer 102. Therefore, it may be possible to detect the cardiac activity from the accelerometer 102 during the monitoring of the sleeping quality in the subject person P. Moreover, the embodiment may further associate a relationship of changes of heart rate of the subject person and sleep event. Furthermore, the detection of the cardiac activity may be performed in a same way as in the method for monitoring the sleeping disorder in the subject person P. In this case, the association may be made between the changes of heart rate and the silence events. However, detecting of the cardiac activity may be performed in a same way in both the monitoring of a sleeping disorder and quality.

Figure 4 shows a schematic view of a configuration of the system for monitoring sleeping disorder and quality in a subject person. In this embodiment described in the Figure 4, the system 4 comprises a mobile device 10, a fixation system 2 and a pulse oximeter 3. Although shown as a wireless pulse oximeter for in the Figure 4, the pulse oximeter 3 may be wired or wireless and is configured to measure oxygen saturation levels of the subject person P. The mobile device 10 of the system 4 comprises a microphone 101 configured to acquire audio signals A indicative of breathing of the subject person P and an accelerometer 102 configured to acquire accelerometer signals including position, angular and movement information of the subject person P. The system 4 further comprises the fixation system 2 as described in the Figure 3.

In further embodiments of the present invention different multimodal features can be extracted from the audio, the accelerometer and the pulse oximeter signals. In such embodiments there is provided a method for monitoring a sleeping quality in a subject person, the method using a mobile device and comprising: collocating the mobile device with the subject person; acquiring audio signals indicative of breathing of the subject person by means of a microphone of the mobile device; acquiring accelerometer signals including position, angular and movement information of the subject person by means of an accelerometer of the mobile device; measuring thoracic movement related to respiration and sleep-disordered breathing by means of the accelerometer of the mobile device; identifying a plurality of sleep events by means of the audio signals; identifying body position at which the sleep event is produced based on the angular information; labelling of the sleep events by multimodal features; and automatic detecting of the multimodal sleep features, wherein the multimodal features are the acquired audio signals, the acquired accelerometer signals and oxygen saturation levels acquired by means of a wireless pulse oximeter via a wireless link to the mobile device.

In such embodiments the detection of multimodal features may be automatic because it can be automatically calculated by custom-made computational algorithms that may not require manual review or annotation of the signals. Several features can be extracted, including features describing the desaturation events (e.g., depth or duration of the desaturation), features related to the apneas and hypopneas (indices counting the number of apneas and/or hypopneas, duration, whether they include snoring...), snoring (from the audio signals or vibration from the accelerometer signals), features related to movement, sleep position or sleep stages, related to cardiac activity, and the like. It is noted that to "label" the sleep event should be understood in that a sleep event is annotated in the sense of identifying and classify it, e.g. in order to indicate that there is an event starting at, say, a time=1100s and ending at time=1122s and that it corresponds to an obstructive apnea. Further, "multimodal features" are to be understood as more general and "multimodal sleep features" may indicate that they are related to aspects of sleep.

Although detailed embodiments have been described, these only serve to provide a better understanding of the invention defined by the independent claims and are not to be seen as limiting.

### Reference Signs

- A: audio signals
- P, P': subject person
- 10: mobile device
- 101: microphone
- 102: accelerometer
- 2: fixation system
- 21: elastic band
- 22: bag
- 3: pulse oximeter
- 4: system

### Clauses

The present application comprises the following clauses:
1. A method for monitoring a sleeping disorder in a subject person, the method using a mobile device and comprising:
   collocating the mobile device with the subject person;
   acquiring audio signals indicative of breathing of the subject person by means of a microphone of the mobile device;
   acquiring accelerometer signals, by means of an accelerometer of the mobile device, to derive position, angular and movement information of the subject person from the accelerometer signals;
   measuring thoracic movement related to respiration and sleep-disordered breathing by means of the accelerometer;
   identifying a plurality of silence events based on the audio signals;
   classifying the silence events into apnea or hypopnea;
   identifying an apnea type of the classified apnea based on the thoracic movement; and
   associating to a classified silence event the respective angular information by combining the audio signals and the accelerometer signals.
2. The method for monitoring a sleeping disorder in a subject person according to clause 1, wherein the method further comprising:
   quantifying sleep movements and sleep position variability based on the accelerometer signals; and
   detecting breathing route during sleep from the audio signals.
3. The method for monitoring a sleeping disorder in a subject person according to any of preceding clause, wherein the method further comprising:
   acquiring oxygen saturation levels of the subject person by means of a wireless pulse oximeter via a wireless link to the mobile device; and
   selecting regions of interest, wherein the regions in interest are regions of the audio signals that are followed by oxygen desaturations measured by the wireless pulse oximeter.
4. The method for monitoring a sleeping disorder in a subject person according to any of preceding clause, wherein the method further comprising:
   removing artefacts by considering the accelerometer signals while identifying the silence events.
5. The method for monitoring a sleeping disorder in a subject person according to any of preceding clause, wherein the method further comprising:
   classifying the silence events into apnea or hypopnea depending on existence of low-intensity respiratory sounds in the audio signals.
6. The method for monitoring a sleeping disorder in a subject person according to any of preceding clause, wherein the method further comprising:
   providing the classified silence events with associated the respective angular information on the mobile device.
7. The method for monitoring a sleeping disorder in a subject person according to any of preceding clause, wherein the method further comprising:
   outputting vibration and/or sound from the mobile device based on the angular information and the oxygen saturation level and/or the audio signals for positional treatment.
8. The method for monitoring a sleeping disorder in a subject person according to any of preceding clause, wherein
   the apnea type is obstructive, central, or mixed;
   the breathing route is oral or nasal; and
   the angular information is one angle, a plurality of angles, or triaxial angles.
9. The method for monitoring a sleeping disorder in a subject person according to any preceding clause, wherein the method further comprising:
   detecting cardiac activity from the accelerometer; and
   associating a relationship of changes of heart rate of the subject person and silence events.
10. A method for monitoring a sleeping quality in a subject person, wherein the method using a mobile device and comprising:
   collocating the mobile device with the subject person;
   acquiring audio signals indicative of breathing of the subject person by means of a microphone of the mobile device;
   acquiring accelerometer signals, by means of an accelerometer of the mobile device, to derive position, angular and movement information of the subject person from the accelerometer signals;
   measuring thoracic movement related to respiration and sleep-disordered breathing by means of the accelerometer;
   identifying a sleep event, including snoring episodes, based on the audio signals; and
   identifying body position at which the sleep event is produced based on the angular information.
11. The method for monitoring a sleeping quality in a subject person according to clause10, wherein the identifying of a sleep event includes identifying a snoring episode.
12. The method for monitoring a sleeping quality in a subject person according to clause10 or 11, wherein the method further comprising:
   automatic detecting of multimodal features of sleep episodes;
      and
   labelling of the sleep event by the multimodal features, wherein
   the multimodal features are derived from the audio signals, the accelerometer signals and oxygen saturation levels acquired by means of a wireless pulse oximeter via a wireless link to the mobile device.
13. The method for monitoring a sleeping quality in a subject person according to any one of clauses10 to 12, wherein the method further comprising:
   detecting cardiac activity from the accelerometer; and
   associating a relationship of changes of heart rate of the subject person and sleep event.
14. A system for monitoring sleeping disorder and quality in a subject person, the system comprising:
   a mobile device comprising:
      a microphone configured to acquire audio signals indicative of breathing of the subject person; and
      an accelerometer configured to acquire accelerometer signals including position, angular and movement information of the subject person;
   a fixation system for collocating the mobile device with the subject person; and
   a pulse oximeter configured to measure oxygen saturation levels of the subject person, wherein
   the fixation system is composed of an elastic band and a bag to place the mobile device.

## Claims

1. A system for monitoring sleeping quality in a subject person, the system comprising:
a mobile device comprising:
a microphone configured to acquire audio signals indicative of breathing of the subject person; and
an accelerometer configured to acquire accelerometer signals including position, angular and movement information of the subject person;
a fixation system for collocating the mobile device with the subject person; and
a pulse oximeter configured to measure oxygen saturation levels of the subject person, wherein
the fixation system is composed of an elastic band and a bag to place the mobile device;
wherein the system is configured for monitoring sleeping quality in a subject person by:
- collocating the mobile device with the subject person;
- acquiring audio signals indicative of breathing of the subject person by means of the microphone;
- acquiring accelerometer signals, by means of the accelerometer, to derive position, angular and movement information of the subject person from the accelerometer signals;
- measuring thoracic movement related to respiration and sleep-disordered breathing by means of the accelerometer;
- identifying a sleep event, including snoring episodes, based on the audio signals; and
- identifying body position at which the sleep event is produced based on the angular information.

2. The system according to claim 1, wherein the identifying of a sleep event includes identifying a snoring episode.

3. The system according to claim 1 or 2, wherein the system is configured for monitoring sleeping quality in a subject person by further:
- automatic detecting of multimodal features of sleep episodes; and
- labelling of the sleep event by the multimodal features, wherein the multimodal features are derived from the audio signals, the accelerometer signals and oxygen saturation levels acquired by means of a wireless pulse oximeter via a wireless link to the mobile device.

4. The system according to claim 3, wherein the multimodal features are one or more selected from the list comprising: features describing the desaturation events, features related to the apneas and hypopneas, snoring, features related to movement, features related to sleep position, features related to sleep stages, or features related to cardiac activity.

5. The system according to any one of claims 1 to 4, wherein the system is configured for monitoring sleeping quality in a subject person by further:
- detecting cardiac activity from the accelerometer; and
- associating a relationship of changes of heart rate of the subject person and sleep event.

6. A method for monitoring a sleeping quality in a subject person, wherein the method using a mobile device and comprising:
collocating the mobile device with the subject person;
acquiring audio signals indicative of breathing of the subject person by means of a microphone of the mobile device;
acquiring accelerometer signals, by means of an accelerometer of the mobile device, to derive position, angular and movement information of the subject person from the accelerometer signals;
measuring thoracic movement related to respiration and sleep-disordered breathing by means of the accelerometer;
identifying a sleep event, including snoring episodes, based on the audio signals; and
identifying body position at which the sleep event is produced based on the angular information.

7. The method for monitoring a sleeping quality in a subject person according to claim 6, wherein the identifying of a sleep event includes identifying a snoring episode.

8. The method for monitoring a sleeping quality in a subject person according to claim 6 or 7, wherein the method further comprising:
automatic detecting of multimodal features of sleep episodes;
and
labelling of the sleep event by the multimodal features, wherein
the multimodal features are derived from the audio signals, the accelerometer signals and oxygen saturation levels acquired by means of a wireless pulse oximeter via a wireless link to the mobile device.

9. The method for monitoring a sleeping quality in a subject person according to claim 8, wherein the multimodal features are one or more selected from the list comprising: features describing the desaturation events, features related to the apneas and hypopneas, snoring, features related to movement, features related to sleep position, features related to sleep stages, or features related to cardiac activity.

10. The method for monitoring a sleeping quality in a subject person according to any one of claims 6 to 9, wherein the method further comprising:
detecting cardiac activity from the accelerometer; and
associating a relationship of changes of heart rate of the subject person and sleep event.
